Europäisches Patentamt

European Patent Office

Office européen des brevets

(11) Veröffentlichungsnummer **0 035 726**
**B1**

(12) # EUROPÄISCHE PATENTSCHRIFT

(45) Veröffentlichungstag der Patentschrift: 28.12.83

(51) Int. Cl.³: **G 01 N 33/92**

(21) Anmeldenummer: 81101471.1

(22) Anmeldetag: 02.03.81

(54) **Verfahren und diagnostisches Mittel zur direkten Bestimmung des Lipidgehaltes der beta-Lipoproteine des Blutes.**

(30) Priorität: 08.03.80 DE 3009037

(43) Veröffentlichungstag der Anmeldung:
16.09.81 Patentblatt 81/37

(45) Bekanntmachung des Hinweises auf die Patenterteilung:
28.12.83 Patentblatt 83/52

(84) Benannte Vertragsstaaten:
AT BE CH DE FR GB IT LI LU NL SE

(56) Entgegenhaltungen:
DE - A - 2 600 664
DE - A - 2 743 524
US - A - 3 645 692

CHEMICAL ABSTRACTS, Band 86, Nr. 21, 23.
Mai 1977, Seite 194, Nr. 152271c Columbus,
Ohio, U.S.A. C.C. HEUCK et al.: "Beta-lipoprotein
cholesterol quantitation with polycations"

(73) Patentinhaber: BOEHRINGER MANNHEIM GMBH
Patentabteilung, Abt. E Sandhofer Strasse 112-
132 Postfach 31 01 20
D-6800 Mannheim 31 Waldhof (DE)

(72) Erfinder: Maier, Josef
Schmädlstrasse 15
D-8120 Weilheim (DE)
Erfinder: Gloger, Manfred, Dr. rer. nat.
Karwendelstrasse 6
D-8120 Weilheim (DE)
Erfinder: Draeger, Brigitte, Dr. rer. nat.
Sprungleitenweg 2
D-8132 Tutzing (DE)

(56) Entgegenhaltungen:
CHEMICAL ABSTRACTS, Band 87, Nr. 21, 21.
November 1977, Seite 215, Nr. 163627v
Columbus, Ohio, U.S.A. C.C. HEUCK et al.:
"Improved direct determination of serum
cholesterol in low-density lipoproteins with use
of polycations"

Courier Press, Leamington Spa, England.

Verfahren und diagnostisches Mittel zur direkten Bestimmung des Lipidgehaltes der $\beta$-Lipoproteine des Blutes

Die Erfindung betrifft ein Verfahren zur direkten Bestimmung des Lipidgehalts der $\beta$-Lipoproteine des Blutes durch selektive Abtrennung der Lipoproteinfraktion aus Blutplasma oder -serum, wobei das Plasma oder Serum mit Polykationen versetzt wird und die aus den Polykationen und den Lipoproteinen gebildeten Komplexe fraktionsweise aus der Lösung entfernt werden.

Ein solches Verfahren ist aus der DE—A—26 00 664 bekannt, bei welchem dem Serum schwache, in Lösung befindliche Polykationen, vorzugsweise in Form einer 40%igen Polyäthylenimin (PEI)-Lösung, zugesetzt werden, wobei sich lösliche Lipoprotein-PEI-Komplexe bilden. Diese löslichen Lipoprotein-PEI-Komplexe werden dadurch selektiv aus der Lösung entfernt, daß man das zuvor mit Polyäthyleniminlösung versetzte Serum mit einem schwachsauren Kationenaustauscher, beispielsweise Methylacrylsäure-Divinylbenzol-Copolymerisat in H-Form, schüttelt und den Überstand dekantiert. Eine selektive Auftrennung der drei Lipoproteinfraktionen, nämlich der $\beta$-Lipoproteine (Low Density-Lipoproteine = LDL) einerseits und der prä-$\beta$-Lipoproteine (Very Low Density-Lipoproteine = VLDL) und $\alpha$-Lipoproteine (High Density-Lipoproteine = HDL) andererseits, wird dadurch erreicht, daß die löslichen Komplexe aus $\beta$-Lipoprotein und PEI überraschenderweise frei in Lösung bleiben, während die löslichen Komplexe aus prä-$\beta$-Lipoprotein und PEI und die löslichen Komplexe aus $\alpha$-Lipoprotein und PEI an den schwachsauren Kationenaustauscher adsorbiert werden. Der Lipidgehalt des dekantierten Überstandes wird dann nach bekannten Verfahren bestimmt; der Cholesteringehalt der im dekantierten Überstand enthaltenen LDL-Fraktion, der dem Gehalt an $\beta$-Cholesterin im Serum entspricht, wird chemisch nach dem Verfahren von Liebermann-Burchard oder enzymatisch bestimmt.

Dieses aus der DE—A 26 00 664 bekannte Verfahren liefert zwar reproduzierbare Meßergebnisse, die im Rahmen einer Fehlergrenze von $\pm$ 10% mit den Referenzwerten der Ultrazentrifugenmethode (Auftrennung der drei Lipoproteinfraktionen nach Dichtegradienten in der Ultrazentrifuge, vgl. Med. Labor. *30*, Heft 12, S. 294 bis 301 (1977)) übereinstimmen, seine klinische Anwendbarkeit wird jedoch dadurch begrenzt, daß es sich um ein vergleichsweise zeitraubendes und langsames Verfahren handelt, das den Einsatz geschulter Fachkräfte erforderlich macht. Es kommt noch hinzu, daß der Zusatz von Hilfs-Ionenaustauschern—neben dem zur Komplexbildung benötigten Polykation—dieGefahr von Verunreinigungen sowie die Gefahr mit sich bringt, daß ein Teil der im Überstand befindlichen LDL-Fraktion beim zwangsläufig erforderlichen Dekantieren, Auswaschen und Filtrieren verlorengeht. Aus diesen Gründen ist das bekannte Verfahren wenig geeignet, als standardisiertes Routineverfahren ohne besonderen labormäßigen Aufwand auch von ungelernten Kräften schnell und sicher ausgeführt zu werden.

Aus der DE—A—27 08 912 ist ein weiteres Verfahren zur selektiven Abtrennung von Lipoproteinen aus Blutplasma oder -serum bekannt, bei dem das Plasma oder Serum zunächst mit bivalenten Kationen, beispielsweise $Mg^{2+}$-, $Ca^{2+}$- oder $Mn^{2+}$-Ionen, und anschließend mit einem Kationenaustauscher versetzt wird, der als Festionen Sulfationen und als Gegenionen ebenfalls bivalente Kationen wie $Mg^{2+}$-, $Ca^{2+}$- oder $Mn^{2+}$-Ionen enthält. Die Gerüstmasse des Kationenaustauschers besteht aus einem vernetzten Kohlenhydrat, einem vernetzten Polysaccharid oder vernetzter Cellulose, die gegebenenfalls mit niedrigen Hydroxyalkylgruppen modifiziert sein können. Dieses Verfahren läßt sich zwar im Gegensatz zu dem eingangs genannten bekannten Verfahren schnell, reproduzierbar und wirtschaftlich in einem Säulenverfahren anwenden, hat aber den Nachteil, daß die LDL- und VLDL-Lipoproteinfraktionen stets gemeinsam an den Kationenaustauscher adsorbiert werden, während die $\alpha$-Lipoproteine im Eluat der ersten Säule verbleiben. Die Bestimmung des Lipidgehaltes der $\beta$-Lipoproteine, also der LDL-Fraktion alleine, ist mit diesem Verfahren nicht möglich.

Der Erfindung liegt die Aufgabe yugrunde, ein Verfahren der eingangs genannten Gattung zu schaffen, das sich schnell, reproduzierbar und wirtschaftlich in einem Säulenverfahren anwenden läßt, dabei aber die oben erläuterten Nachteile nicht aufweist, sowie ein diagnostisches Mittel zur Durchführung dieses Verfahrens zu schaffen.

Diese Aufgabe wird gemäß der Erfindung bei einem Verfahren zur direkten Bestimmung des Lipidgehalts der $\beta$-Lipoproteine des Blutes durch selektive Abtrennung der Lipoproteinfraktionen aus Blutplasma oder -serum, wobei das Plasma oder Serum mit Polykationen versetzt wird und die aus den Polykationen und den Lipoproteinen gebildeten Komplexe fraktionsweise aus der Lösung entfernt werden, dadurch gelöst, daß man prä-$\beta$-Lipoproteine (VLDL) und $\alpha$-Lipoproteine (HDL) durch Versetzen des Plasmas oder Serums mit einem festen, wasserunlöslichen Anionenaustauscher aus einem quaternäre Ammoniumgruppen als Festionen enthaltenden Dextran oder aus vernetztem Polyäthylenimin (PEI) wobei gegebenenfalls in dessen polymere Matrix Dextrane eingebunden sind, abtrennt und den Lipidgehalt der in Lösung verbleibenden $\beta$-Lipoproteine (LDL) nach bekannten Methoden mißt.

Mit dem erfindungsgemäßen Verfahren wird erreicht, daß der zu untersuchenden Körperflüssigkeit weder eine wäßrige Reagenslösung zugesetzt werden muß, noch in die Körperflüssigkeit ein fester, pulver- oder granulatförmiger Hilfs-Ionenaustauscher eingerührt werden muß, so daß alle Schwierigkeiten, die bei den früher bekannten Verfahren im Zusammenhang mit der Abtrennung der Hilfs-Ionenaustauscher oder dem Versetzen der zu untersuchenden Flüssigkeit mit Fremdlösungen auftraten, vermieden werden. Bei dem Erfindungsgemäßen Verfahren werden die Funktionen des früher in Form wäßriger Lösungen zugesetzten Polyäthylenimins, nämlich die Komplexbildung mit den Lipoproteinen und die Funktionen des früher gesondert zugesetzten Hilfs- Ionenaustauschers, nämlich die selektive Adsorption der Lipoprotein-PEI-Komplexe, in einem einzigen Schritt vereinigt, wobei weder irgendwelche Reagenslösungen hergestellt, gereinigt oder abgetrennt werden müssen, noch ein Dekantieren, Filtrieren oder Reinigen des Eluats erforderlich ist. Der erfindungsgemäß verwendete feste Anionenaustauscher braucht nur in eine Säule gefüllt, und die zu untersuchende Körperflüssigkeit in herkömmlicher Weise chromatographiert zu werden, wobei die LDL-Fraktion ungehindert hindurchläuft, während die VLDL- und die HDL-Fraktionen an den unlöslichen Träger adsorbiert werden.

Vorteilhafte Weiterbildungen des erfindungsgemäßen Verfahrens ergeben sich aus den Beispielen in Verbindung mit den Unteransprüchen.

Vorteilhaft ist die Verwendung von mit bifunktionellen Epoxidverbindungen, vorzugsweise Epichlorhydrin, oder von mit Dialdehyden, die von Dicarbonsäuren mit 3 bis 6 Kohlenstoffatomen abgeleitet sind, vorzugsweise von mit Glutardialdehyd, vernetztem PEI. Die Vernetzung des PEI erfolgt dabei nach beispielsweise aus der US—A—3 796 634, der DE—C—1 168 078 oder der DE—B—1 056 825 bekannten Verfahren. Epichlorhydrin wird dabei vorzugsweise in einer Menge von 2,5 bis 3,5 ml zur Vernetzung von je 21,6 ml (20 g) einer 50%-igen PEI-Lösung verwendet.

Bei der Verwendung des wasserunlöslichen, vernetzten PEI zur Abtrennung der VLDL- und HDL-Fraktionen gelangen je nach Art und Provenienz der zu untersuchenden Probe etwa 5 bis 10% des HDL-Anteils im Serum bzw. im Plasma in den Durchlauf zur LDL-Fraktion. Dieser Anteil ist jedoch so gering, daß er sich nur elektrophoretisch nachweisen läßt.

Es wurde jedoch gefunden, daß auch der 5 bis 10%ige HDL-Anteil im Eluat vermieden werden kann, wenn die Vernetzung des PEI in Gegenwart von porösen keramischen Materialien oder porösen Gläsern mit definierter Porengröße, sogenannten "controlled-pore-glasses", durchgeführt wird. Als poröse keramische Materialien eignen sich hierfür gebrannter Ton, Kieselgur und Diatomeenerde sowie die auf Grundlage dieser Materialien hergestellten, jedoch mit den verschiedensten Polymeren vermischten, auf dem Markt erhältlichen Produkte, während sich als CPG-Gläser solche mit einem mittleren Porendurchmesser von 17,5 bis 25,0 nm als optimal erwiesen haben.

Die Verwendung von in Gegenwart von keramischen Materialien oder CPG-Gläsern vernetztem PEI has außerdem den Vorteil, daß ein unter Umständen auftretendes störendes Quellverhalten von wenig vernetzten PEI-Gelen sicher vermieden wird.

Insbesondere bei trüben Seren kann es auch bei Anwendung des erfindungsgemäßen Verfahrens vorkommen, daß ein geringer Anteil der VLDL-Fraktion in den Durchlauf zum LDL gelangt. Es wurde jedoch gefunden, daß dies dadurch vermieden werden kann, daß das verwendete vernetzte Polyäthylenimin mit hydrophoben Resten, insbesondere hydrophoben Aminen, bei der Vernetzung modifiziert wird. Durch diese Modifizierung mit solchen hydrophoben Resten wird die Bindungskapazität für VLDL-Lipoproteine stark erhöht. Geeignete hydropobe Amine sind primäre geradkettige oder verzweigte Alkylamine mit 3 bis 12 Kohlenstoffatomen, beispielsweise Propylamin, n-Butylamin, tert.-Butylamin, Hexylamin, Dodecylamin, N-Hydroxymethyl-äthylenimin, 3-Amino-2,2-dimethylpropan-1-ol, N,N-2-Tetramethylpropan-1,3-diamin. Diese Amine werden als Comonomere bei der Vernetzung des PEI mit bifunktionellen Epoxidverbindungen oder Dialdehyden eingesetzt.

Ganz allgemein hähgt der Grad der Auftrennung der Einzelnen Lipoproteinfraktionen an der Säule sowohl von der Ionenstärke der Lösung als auch vom pH-Wert und dem Vernetzungsgrad der PEI-Matrix ab. Mit steigender Vernetzung nimmt die Bindungsfähigkeit der PEI-Matrix für Lipoproteine ab.

Es hat sich weiter gezeigt, daß sich solche Anionenaustauscher besonders vorteilhaft zur Abtrennung der prä-$\beta$-Lipoproteine und $\alpha$-Lipoproteine aus dem Plasma oder Serum verwenden lassen, in deren polymere Matrix Dextrane, vorzugsweise solche mit einem mittleren Molekulargewicht von 20 000 bis 800 000, eingebunden sind. Die Dextrane werden dabei zweckmäßig während der Vernetzung des Anionenaustauschers, insbesondere des Polyäthylenimins, mit bifunktionellen Epoxidverbindungen oder mit Dialdehyden der Lösung des noch unvernetzten Anionenaustauschers zugegeben, so daß die Dextran-Einheiten während der Vernetzung in die polymere Matrix mit eingebunden werden.

Gegenstand der Erfindung ist weiter ein diagnostisches Mittel zur Durchführung des erfindungsgemäßen Verfahrens, das einen der erfindungsgemäß verwendeten Anionenaustauscher enthält. Vorzugsweise enthält das diagnostische Mittel in Form eines Test-Sets oder einer Test-Kombination alle erforderlichen Reagenzien, die jeweils einzeln in einer für eine Bestimmung oder eine Reihe von Bestimmungen ausreichenden Menge abgepackt sind. Das Test-Set kann auch bereits eine Testsäule enthalten. Beispielsweise kann das erfindungsgemäße diagnostische Mittel bestehen aus

(a) einer Testsäule aus durchsichtigem oder durchscheinendem Material,

(b) einer für die Füllung der Testsäule ausreichenden Menge an einem der gemäß einem der Ansprüche 1 bis 13 verwendeten Anionenaustauscher,

(c) einer gesondert abgepackten geeigneten Pufferlösung, pH 6,5 bis 7,5, für die Elution der Lipoprotein- fraktionen und

(d) reiner, ebenfalls gesondert abgepackter Kochsalzlösung.

Im folgenden wird beispielhaft die Herstellung von erfindungsgemäß verwendbaren Anionenaustauschern beschrieben:

3

*Anionenaustauscher 1:* PEI-Epichlorhydrin-Chromosorb®

20 g 50%-ige Polyäthylenimin-Lösung (G 35, BASF) werden in 38 ml 0,5 m-NaOH gelöst und mit 2,5 ml Epichlorhydrin vernetzt. Der Ansatz wird 15 min kräftig gerührt, zur Beschichtung in ca. 50 g Chromosorb® (eingetragenes Warenzeichen der Johns-Manville International Corporation, New York; im Handel befindliches Trägermaterial auf Diatomeenerde-Basis) eingerührt und 3 h bei 60°C nacherhitzt. Der Anionenaustauscher wird in einem Prüfsieb (Maschenweite 0,5 mm) zerkleinert, von Feinstteilen befreit, ausreichend gewaschen und mit 0,33 NaCl-0,05 m-Imidazol-Salzsäure-Puffer, pH 7,2, vorequilibriert.

*Anionenaustauscher 2:* PEI-Epichlorhydrin-Comonomere-Chromosorb®

Die Vernetzung des PEI mit Epichlorhydrin erfolgt, wie bei der Herstellung des Anionenaustauschers 1 beschrieben, jedoch in Gegenwart der nachstehend genannten Comonomeren, unter Verwendung des folgenden Ansatzes:

10 g PEI (50%-ige Lösung),
20 ml 0,5 m-NaOH,
5 ml Dioxan, sowie entweder
0,2 g Butylamin, oder
0,2 g Propylamin, oder
0,2 g Hexylamin, oder
0,2 g Dodecylamin, oder
0,5 g N-Hydroxymethyl-äthylenimin, oder
2 g 3-Amino-2,2-dimethylpropan-1-ol (BASF) oder
1,5 g N,N-2-Tetramethylpropan-1,3-diamin.

Aufarbeitung wie bei der Herstellung des Anionenaustauschers 1 beschrieben.

*Anionenaustauscher 3:* PEI-Glutardialdehyd-CPG-Glas oder Spherosil®

5 g CPG-Glas mit einem mittleren Porendurchmesser von 25nm (Hersteller: Electro Nucleonics) werden mit Glutardialdehyd-Lösung benetzt und unter leichtem Rühren mit einer 10%igen PEI-Lösung, pH 8,0, zur Reaktion gebracht. Nach 30 min wird abdekantiert und die beschichteten Glaspartikel werden mit 0,1 N-HCl, 0,1 N-NaOH und Wasser gründlich gewaschen.

*Anionenaustauscher 4:* PEI-Epichlorhydrin-CPG-Glas

Ein Gemisch aus 20 g PEI—G 35, 50%ig (BASF), 45 ml 0,5 m-NaOH und 2,5 ml Epichlorhydrin wird 2 h lang bei Raumtemperatur kräftig gerüht. 70 g CPG-Glas mit einem mittleren Porendurchmesser von 17,7 nm (Hersteller: Electro Nucleonics) werden mit dieser Lösung benetzt und 2 h bei 70° gehalten. Die beschichteten Glaspartikel werden in eine Säule gefüllt, mit 21 1 m-HCl, 51 0,5 m-Phosphatpuffer, pH 7,4, und anschließend mit der gleichen Menge aqua bidestillata gewaschen. Für die Auftrennung der Lipoproteine wird der Anionenaustauscher mit 0,25 m-NaCl-0,05 m-Imidazol-HCl-Puffer, pH 7,2, oder mit 0,42 m-KCl vorequilibriert.

*Anionenaustauscher 5:* PEI/Dextran-Epichlorhydrin-CPG-Glas

Ein Gemisch aus 20 g PEI—G 35, 50%ig (BASF), 45 ml 0,5 m-NaOH, 1 g Dextran T500 (Hersteller: Pharmacia mittleres Molekulargewicht: 500 000) und 2,5 ml Epichlorhydrin wird 2 h lang bei Raumtemperatur kräftig gerührt. 56 g CPG-Glas mit einem mittleren Porendurchmesser von 20 nm werden mit dieser Lösung benetzt und 2 h bei 70°C gehalten. Die beschichteten Glaspartikel werden, wie beim Anionenaustauscher 4 beschrieben, gewaschen und vorequilibriert.

*Anionenaustauscher 6:* PEI/Dextran-Epichlorhydrin-CPG-Glas

Ein Gemisch aus 20 g PEI—G 35, 50%ig (BASF), 45 ml 0,5 m-NaOH, 1 g Dextran T70 (mittleres Molekulargewicht: 70 000) und 2,5 ml Epichlorhydrin wird 2 h lang bei Raumtemperatur kräftig gerührt. 56 g CPG-Glas mit einem mittleren Porendurchmesser von 20 nm werden mit dieser Lösung benetzt und 2 h bei 70°C gehalten. Die beschichteten Glaspartikel werden, wie vorstehend beschrieben, gewaschen und vorequilibriert.

*Anionenaustauscher 7:* PEI/Dextran-Epichlorhydrin-CPG-Glas

Ein Gemisch aus 20 g PEI—G 35, 50%ig (BASF), 45 ml 0,5 m-NaOH, 1 g Dextran T40 (mittleres Molekulargewicht: 40 000) und 2,5 ml Epichlorhydrin wird 2 h lang bei Raumtemperatur kräftig gerührt. 56 g CPG-Glas mit einem mittleren Porendurchmesser von 20 nm werden mit dieser Lösung benetzt und 2 h bei 70°C gehalten. Die beschichteten Glaspartikel werden wieder, wie vorstehend beschrieben, gewaschen und vorequilibriert.

*Anionenaustauscher 8:*

Diäthyl-2-hydroxypropyl-ammonium-Gruppen enthaltendes Dextrangel (QAE-Sephadex®, Hersteller: Pharmacia) wird mit destilliertem Wasser vorgequollen und anschließend mit 0,11 m-NaCl erschöpfend equilibriert und anschließend in eine Säule gefüllt.

Beispiel

Bestimmung des Lipidgehalts der β-Lipoproteine des Blutes:

0,05 bis 0,15 ml des zu untersuchenden Humanserums werden auf eine Säule (5 × 100 mm) aufgetragen, die mit 2 ml von einem der vorstehend beschriebenen Anionenaustauscher 1 bis 8 gefüllt und mit 0,25 m-NaCl-0,05 m-Imidazol-Salzsäure-Pufferlösung, pH 7,2, oder mit 0,42 m-KCl-Lösung voreaquilibriert ist. Es wird mit 4 ml des genannten Puffers oder mit 2 ml 0,11 m-NaCl-Lösung eluiert und der LDL-Cholesterin-Gehalt im Eluat nach einer der folgenden bekannten analytischen Methoden bestimmt:

1. Lipoprotein-Elektrophorese (P. Müller et al.: Lab. med. *1*, 145—148 (1977));

2. Cholesterinbestimmung, Testkombination der Boehringer Mannheim GmbH (P. Röschlau et al.: Z. Klin. Chem. Klin. Biochem. *12* 403 (1974));.

3. LDL-Cholesterin nach Friedewald (W. Friedewald et al.: Clin. Chem. *18* 499 (1972)).

Die Abschätzung des Gehalts an LDL-Cholesterin erfolgt gemäß folgender Formel (Friedewald):

$$C_{LDL} = C_{Serum} - (C_{HDL} + \frac{TG}{5})$$

mit C = Cholesterinkonzentration in mg/100 ml und TG = Konzentration an Triglyceriden in mg/100 ml.

Die an den Anionenaustauscher adsorbierten Lipoproteine (VLDL- und HDL-Fraktionen) werden mit NaCl-Lösung eluiert, und das HDL-Cholesterin wird nach Fällung der VLDL-Fraktion mit Phosphowolframsäure oder Heparin im überstand nach einer der bekannten Methoden bestimmt.

In der folgenden Tabelle 1 ist für verschiedene Proben der jeweilige Gesamtcholesteringehalt im Serum, dem nach der Methode von Friedewald gemessenen Cholesteringehalt der LDL-Fraktion und dem erfindungsgemäß gemessenen Cholesteringehalt der LDL-Fraktion, jeweils gemessen in mg/100 ml, gegenübergestellt. Dabei zeigt sich, daß die erfindungsgemäß gemessenen Werte sehr gut mit den nach der Methode von Friedewald ermittelten Werten übereinstimmen.

| Vergleich | | Erfindungsgemäß | | |
|---|---|---|---|---|
| Chol.Serum | Chol.LDL (Friedewald) | Chol.LDL (Anionenaust.1) | Chol.LDL (Anionenaust.5) | Chol.LDL (Anionenaust.8) |
| 156 | 105 | | 100 | |
| 163 | 102 | | | 109 |
| 165 | 101 | | 92 | |
| 176 | 114 | | 106 | |
| 179 | 110 | | 115 | |
| 180 | 115 | 118 | | |
| 183 | 126 | 121 | | |
| 196 | 114 | | | 122 |
| 198 | 131 | 135 | | |
| 205 | 146 | 138 | | |
| 206 | 126 | | 117 | |
| 209 | 129 | 136 | | |
| 215 | 121 | | 136 | |
| 226 | 156 | | | 146 |
| 230 | 168 | 159 | | |
| 245 | 171 | 185 | | |
| 249 | 161 | 163 | | |
| 262 | 181 | | | 173 |
| 271 | 172 | | | 176 |
| 278 | 194 | 210 | | |
| 281 | 198 | 182 | | |
| 285 | 205 | 198 | | |
| 295 | 209 | 203 | | |
| 309 | 211 | 221 | | |
| 315 | 216 | 224 | | |
| 342 | 209 | 219 | | |
| 364 | 245 | 263 | | |

TAB. 1

Tabelle 2 gibt die Präzision des erfindungsgemäßen Verfahrens durch Angabe des Variationskoeffizienten und der Wiederfindung (in %) wieder.

| n | Chol.$_{Serum}$ (mg/100 ml) | Chol.$_{LDL}$ (mg/100 ml) | VK | Wiederfindung (%) |
|----|------|------|------|------|
| 10 | 182 | 110 | 2,70 | 96 |
| 10 | 168 | 95 | 2,31 | 95 |
| 10 | 172 | 116 | 3,10 | 94 |
| 8 | 275 | 190 | 2,38 | 96 |
| 10 | 320 | 219 | 2,59 | 95 |
| 10 | 195 | 132 | 2,18 | 97 |
| 6 | 218 | 157 | 1,19 | 96 |
| 10 | 361 | 246 | 2,56 | 94 |

n = Anzahl der Messungen

VK = Variationskoeffizient

TAB. 2

In Tabelle 3 sind für verschiedene Seren jeweils der Gesamtcholesteringehalt, der Cholesteringehalt der HDL-Fraktion, der Gehalt an Triglyceriden, der erfindungsgemäß ermittelte Cholesteringehalt der LDL-Fraktion unter Verwendung des oben beschriebenen Anionenaust.Nr.4 und der nach Friedewald ermittelte Cholesteringehalt der LDL-Fraktion angegeben.

| Serum lfd.Nr. | Gesamt-Cholesterin | HDL-Chol. | Triglyceride | LDL-Chol. m.Aust.4 (erfind.-gemäß) | LDL-Chol. (n.Frie-dewald) |
|---|---|---|---|---|---|
| | | | mg/100 ml | | |
| 1 | 205 | 41 | 150 | 139 | 134 |
| 2 | 248 | 38 | 175 | 169 | 175 |
| 3 | 174 | 45 | 90 | 108 | 111 |
| 4 (trüb) | 285 | 40 | 330 | 179 | 179 |
| 5 (trüb) | 230 | 30 | 360 | 143 | 128 |
| 6 | 291 | 34 | 174 | 209 | 222 |
| 7 | 184 | 44 | 110 | 112 | 118 |
| 8 | 194 | 38 | 130 | 122 | 130 |
| 9 | 180 | 49 | 95 | 104 | 112 |
| 10 (leicht trüb) | 320 | 45 | 410 | 215 | 193 |
| 11 | 215 | 26 | 160 | 149 | 157 |
| 12 | 203 | 52 | 104 | 124 | 130 |

TAB 3

**Patentansprüche**

1. Verfahren zur direkten Bestimmung des Lipidgehalts der $\beta$-Lipoproteine des Blutes durch selektive Abtrennung der Lipoproteinfraktionen aus Blutplasma oder -serum, wobei das Plasma oder Serum mit Polykationen versetzt wird und die aus den Polykationen und den Lipoproteinen gebildeten Komplexe fraktionsweise aus der Lösung entfernt werden, dadurch gekennzeichnet, daß man prä-$\beta$-Lipoproteine (VLDL) und $\alpha$-Lipoproteine (HDL) durch Versetzen des Plasmas oder Serums mit einem festen, wasserunlöslichen Anionenaustauscher aus einem quaternäre Ammoniumgruppen als Festionen enthaltenden Dextran oder aus vernetztem Polyäthylenimin (PEI) wobei gegebenenfalls in dessen polymere Matrix Dextrane eingebunden sind, abtrennt und den Lipidgehalt der in Lösung verbleibenden $\beta$-Lipoproteine (LDL) nach bekannten Methoden mißt.

2. Verfahren nach Anspruch 1, dadurch gekennzeichnet, daß ein Diäthyl-2-hydroxypropyl-ammonium-gruppen als Festionen enthaltendes Dextran verwendet wird.

3. Verfahren nach Anspruch 1, dadurch gekennzeichnet, daß man zur Abtrennung der VLDL- und HDL-Fraktionen PEI verwendet, das mit bifunktionellen Epoxidverbindungen, vorzugsweise Epichlorhydrin, vernetzt ist.

4. Verfahren nach Anspruch 1, dadurch gekennzeichnet, daß man zur Abtrennung der VLDL- und HDL-Fraktionen PEI verwendet, das mit Dialdehyden mit 3 bis 6 Kohlenstoffatomen, vorzugsweise Glutardialdehyd, vernetzt ist.

5. Verfahren nach Anspruch 3 oder 4, dadurch gekennzeichnet, daß man zur Abtrennung der VLDL- und HDL-Fraktionen PEI verwendet, das mit bifunktionellen Epoxidverbindungen oder Dialdehyden in Gegenwart hydrophober Amine vernetzt ist.

6. Verfahren nach Anspruch 5, dadurch gekennzeichnet, daß man PEI verwendet, das in Gegenwart von primären geradkettigen oder verzweigten Alkylaminen mit 3 bis 12 Kohlenstoffatomen vernetzt ist.

7. Verfahren nach einem der Ansprüche 1 bis 6, dadurch gekennzeichnet, daß man zur Abtrennung der VLDL- und HDL-Fraktionen feinteilige poröse Trägermaterialien verwendet, die mit dem PEI oder Dextran beschichtet oder umhüllt sind.

8. Verfahren nach Anspruch 7, dadurch gekennzeichnet, daß man als feinteilige poröse Trägermaterialien gebrannten Ton, Kieselgur, Diatomeenerde, Gläser mit definierter Porengröße (CPG-Gläser) oder Gemische dieser Trägermaterialien verwendet.

9. Verfahren nach Anspruch 8, dadurch gekennzeichnet, daß man als feinteiliges poröses Trägermaterial CPG-Glas mit einem mittleren Porendurchmesser von 17,5 bis 25 nm verwendet.

10. Verfahren nach Anspruch 1, dadurch gekennzeichnet, daß ein PEI verwendet wird, in dessen polymere Matrix Dextrane mit einem mittleren Molekulargewicht von 20 000 bis 800 000 eingebunden sind.

11. Verfahren nach einem der Ansprüche 1 bis 10, dadurch gekennzeichnet, daß man die VLDL- und HDL-Fraktionen dadurch von der LDL-Fraktionen abtrennt, daß man das Plasma oder Serum auf eine mit dem Dextran oder PEI oder mit dem damit beschichteten Feinteiligen porösen Trägermaterial gefüllte Säule gibt und mit Pufferlösung pH 6,5 bis 7,5 chromatographiert und LDL, VLDL und HDL selektiv eluiert.

12. Verfahren nach Anspruch 11, dadurch gekennzeichnet, daß man als Pufferlösung einen Natriumchlorid-Imidazol-Salzsäure-Puffer zur Elution des LDL verwendet.

13. Verfahren nach Anspruch 12, dadurch gekennzeichnet, daß die NaCl-Konzentration des Puffers nach der Elution des LDL solange schrittweise erhöht wird, bis sich das VLDL im Eluat nachweisen läßt, daß dann bei konstanter NaCl-Konzentration das VLDL vollständig eluiert wird und danach mit reiner Kochsalzlösung das HDL eluiert wird.

14. Diagnostisches Mittel zur Durchführung des Verfahrens gemäß den Ansprüchen 1 bis 13, enthaltend einen der gemäß einem der Ansprüche 1 bis 13 verwenden festen, wasserumlöslichen Anionenaustauscher aus Dextran oder PEI.

15. Diagnostisches Mittel nach Anspruch 14, bestehend aus
(a) einer Testsäule aus durchsichtigem oder durchscheinendem Material,
(b) einer die Füllung der Testsäule ausreichenden Menge an einem der gemäß einem der Ansprüche 1 bis 13 verwendeten festen, wasserumlöslichen Anionenaustauscher aus Dextran oder PEI
(c) einer gesondert abgepackten geeigneten Pufferlösung, pH 6,5 bis 7,5, für die Elution der Lipoproteinfraktionen und
(d) reiner, ebenfalls gesondert abgepackter Kochsalzlösung.

## Revendications

1. Procédé pour le dosage direct de la teneur en lipides des $\beta$-lipoprotéines du sang par séparation sélective des fractions de lipoprotéines du plasma ou du sérum sanguin, dans lequel le plasma ou le sérum est additionné de polycations et les complexes formés à partir des polycations et des lipoprotéines sont éliminés se la solution par fractions, caractérisé en ce qu'on élimine les pré-$\beta$-lipoprotéines (VLDL) et les $\alpha$-lipoprotéines (HDL) en ajoutant au plasma ou au sérum un échangeur d'anions, solide et insoluble dans l'eau, constitué par du dextrane renfermant des groupes d'ammonium quaternaire sous forme d'ions fixes ou constitué par de la polyéthylène-imine (PEI) dans la matrice polymère de laquelle sont éventuellement implantés des dextranes, et on dose la teneur en lipides des $\beta$-lipoprotéines (LDL) restant dans la solution selon un procédé connu.

2. Procédé selon la revendication 1, caractérisé en ce qu'on utilise un dextrane contenant des groupes de diéthyl 2-hydroxypropylammonium comme ions fixes.

3. Procédé selon la revendication 1, caractérisé en ce qu'on utilise pour l'élimination des fractions VLDL et HDL une PEI réticulée par des dérivés d'époxy bifonctionnels, de préférence par l'épichlorhydrine.

4. Procédé selon la revendication 1, caractérisé en ce qu'on utilise pour l'élimination des fractions VLDL et HDL une PEI réticulée par des dialdéhydes ayant de 3 à 6 atomes de carbone, de préférence par du glutardialdéhyde.

5. Procédé selon la revendication 3 ou 4, caractérisé en ce qu'on utilise pour l'élimination des fractions VLDL et HDL une PEI réticulée en présence d'amines hydrophobes, par des dérivés d'époxy bifonctionnels ou des dialdéhydes.

6. Procédé selon la revendication 5, caractérisé en ce qu'on utilise une PEI dont la réticulation a été effectuée en présence d'alkylamines primaires à chaîne droite ou ramifiée ayant de 3 à 12 atomes de carbone.

7. Procédé selon l'une quelconque des revendications 1 à 6, caractérisé en ce qu'on utilise pour l'élimination des fractions VLDL et HDL des matières de support poreuses finement divisées pourvues d'une couche de PEI ou dextrane ou enrobées de ces derniers.

8. Procédé selon la revendication 7, caractérisé en ce qu'on utilise comme matières de support poreuses finement divisées de l'argile calciné, du kieselguhr, de la terre de diatomées, des verres dont la taille des pores est définie (verres CPG) ou des mélanges de telles matières de support.

9. Procédé selon la revendication 8, caractérisé en ce qu'on utilise comme matière de support

## O 035 726

poreuse finement divisée, du verre CPG présentant un diamètre moyen des pores compris entre 17,5 et 25 nm.

10. Procédé selon la revendication 1, caractérisé en ce qu'on utilise une PEI dans la matrice polymère de laquelle sont implantés des dextranes ayant un poids moléculaire moyen compris entre 20 000 et 800 000.

11. Procédé selon l'une des revendications 1 à 10, caractérisé en ce qu'on sépare les fractions VLDL et HDL des fractions LDL en versant le plasma ou le sérum sur une colonne remplie de dextrane ou de PEI ou de la matière de support poreuse finement divisée comportant une couche de dextrane ou de PEI, et on effectue le processus chromatographique avec un tampon dont le pH est compris entre 6,5 et 7,5 et on élue sélectivement le LDL, le VLDL et le HDL.

12. Procédé selon la revendication 11, caractérisé en ce qu'on utilise comme tampon pour l'élution du LDL, un tampon NaCl-imidazole-HCl.

13. Procédé selon la revendication 12, caractérisé en ce que la concentration du NaCl dans le tampon est augmentée graduellement après l'élution du LDL jusqu'à ce que la présence du VLDL puisse être démontrée dans l'élu at, en ce qu'on élue ensuite à concentration constante de NaCl le VLDL complètement, après quoi on élue le HDL avec une solution de NaCl pur.

14. Agent de diagnostic pour la mise en oeuvre du procédé selon les revendications 1 à 13, contenant un échangeur d'anions solide insoluble dans l'eau constitué par du dextrane ou de la PEI, utilisé selon une des revendications 1 à 13.

15. Agent de diagnostic selon la revendication 14 constitué par
a) une colonne de test en matière transparente un translucide
b) une quantité suffisante pour le remplissage de la colonne d'un échangeur d'anions solide insoluble dans l'eau constitué par du dextrane ou de la PEI, utilisé selon une des revendications 1 à 13
c) un tampon approprié séparément emballé dont le pH est compris entre 6, 5 et 7, 5 pour l'élution des fractions de lipoprotéines, et
d) une solution de NaCl pur également emballé séparément.

## Claims

1. Process for the direct determination of the lipid content of the $\beta$-lipoproteins of the blood by selective separation of the lipoprotein fractions from blood plasma or serum, whereby the plasma or serum is mixed with polycations and the complexes formed from the polycations and the lipoproteins are removed fractionally from the solution, characterised in that one separates pre-$\beta$-lipoproteins (VLDL) and $\alpha$-lipoproteins (HDL) by mixing the plasma or serum with a solid, water-insoluble anion exchanger of a dextran containing quaternary ammonium groups as fixed ions or of cross-linked polyethyleneimine (PEI), whereby dextrans are optionally bound in its polymeric matrix, and measures the lipid content of the $\beta$-lipoproteins (LDL) remaining in solution by known methods.

2. Process according to claim 1, characterised in that a dextran containing diethyl-2-hydroxypropylammonium groups as fixed ions is used.

3. Process according to claim 1, characterised in that, for the separation of the VLDL and HDL fractions, one uses PEI which is cross-linked with bifunctional epoxide compounds, preferably epichlorohydrin.

4. Process according to claim 1, characterised in that, for the separation of the VLDL and HDL fractions, one uses PEI which is cross-linked with dialdehydes with 3 to 6 carbon atoms, preferably glutardialdehyde.

5. Process according to claim 3 or 4, characterised in that, for the separation of the VLDL and HDL fractions, one uses PEI which is cross-linked with bifunctional epoxide compounds or dialdehydes in the presence of hydrophobic amines.

6. Process according to claim 5, characterised in that one uses PEI which is cross-linked in the presence of primary straight-chained or branched alkylamines with 3 to 12 carbon atoms.

7. Process according to one of claims 1 to 6, characterised in that, for the separation of the VLDL and HDL fractions, one uses finely-divided porous carrier materials which are coated or covered with the PEI or dextran.

8. Process according to claim 7, characterised in that, as finely-divided porous carrier materials, one uses calcined clay, kieselguhr, diatomaceous earths, glasses with definite pore size (CPG glasses) or mixtures of these carrier materials.

9. Process according to claim 8, characterised in that, as finely-divided porous carrier material, one uses CPG glass with an average pore diameter of 17.5 to 25 nm.

10. Process according to claim 1, characterised in that a PEI is used in the polymeric matrix of which are bound dextrans with an average molecular weight of 20,000 to 800,000.

11. Process according to one of claims 1 to 10, characterised in that one separates the VLDL and HDL fractions from the LDL fractions in that one applies the serum or plasma to a column filled with the dextran or PEI or with finely-divided porous carrier material coated therewith and chromatographs with buffer solution pH 6.5 to 7.5 and selectively elutes LDL, VLDL and HDL.

12. Process according to claim 11, characterised in that, as buffer solution, one uses a sodium chlorideimidazole-hydrochloric acid buffer for the elution of the LDL.

13. Process according to claim 12, characterised in that the NaCl concentrations of the buffer, after the elution of the LDL, is increased stepwise until the VLDL can be detected in the eluate, that the VLDL is then completely eluted at constant NaCl concentration and thereafter the HDL is eluted with pure common salt solution.

14. Diagnostic agent for the carrying out of the process according to claims 1 to 13, containing one of the solid, water-insoluble anion exchangers of dextran or PEI used according to one of claims 1 to 13.

15. Diagnostic agent according to claim 14, consisting of

(a) a test column of transparent or translucent material,

(b) an amount of one of the solid, water-insoluble anion exchangers of dextran or PEI according to one of claims 1 to 13 sufficient for the filling of the test column.

(c) a separately packed, appropriate buffer solution, pH 6.5 to 7.6, for the elution of the lipoprotein fractions and

(d) pure, also separately packed common salt solution.